(19) Europäisches Patentamt
European Patent Office
Office européen des brevets

(11) Veröffentlichungsnummer: **0 530 615 B1**

(12) # EUROPÄISCHE PATENTSCHRIFT

(45) Veröffentlichungstag der Patentschrift: **18.10.95**

(51) Int. Cl.[6]: **C07C 69/96**, C07C 68/06

(21) Anmeldenummer: **92114286.5**

(22) Anmeldetag: **21.08.92**

(54) **Verfahren zur kontinuierlichen Herstellung von Dialkylcarbonaten.**

(30) Priorität: **03.09.91 DE 4129316**

(43) Veröffentlichungstag der Anmeldung:
**10.03.93 Patentblatt 93/10**

(45) Bekanntmachung des Hinweises auf die Patenterteilung:
**18.10.95 Patentblatt 95/42**

(84) Benannte Vertragsstaaten:
**BE CH DE FR GB IT LI NL**

(56) Entgegenhaltungen:
**EP-A- 0 298 167**
**US-A- 3 803 201**

(73) Patentinhaber: **BAYER AG**
**D-51368 Leverkusen (DE)**

(72) Erfinder: **Buysch, Hans-Josef, Dr.**
**Brandenburger Strasse 28**
**W-4150 Krefeld (DE)**
Erfinder: **Klausener, Alexander, Dr.**
**Weissdornweg 37**
**W-5190 Stolberg (DE)**
Erfinder: **Langer, Reinhard, Dr.**
**Scheiblerstrasse 111**
**W-4150 Krefeld (DE)**
Erfinder: **Mais, Franz-Josef, Dr.**
**Gustav-Poensgen-Strasse 23**
**W-4000 Düsseldorf (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein kontinuierliches Verfahren zur Herstellung von Dialkylcarbonaten durch Umesterung von Ethylen- oder Propylencarbonat mit $C_1$-$C_4$-Alkoholen in Gegenwart eines Katalysators, wobei die Ausgangsprodukte im Gegenstrom zueinander geführt werden.

Es ist bekannt, Ethylen- und Propylenglykolcarbonat (Glykolcarbonate) mit Alkoholen in Gegenwart von Katalysatoren zu Dialkylcarbonaten und Ethylenglykol (EG) bzw. Propylenglykol umzusetzen. Obwohl diese Reaktionen mit hoher Selektivität ablaufen können, weisen die Verfahren bei ihrer Durchführung eine Reihe von Nachteilen auf. In der Regel verläuft die Umesterung bei Normaldruck relativ langsam, so daß die Anwendung erhöhter Temperatur, vielfach oberhalb des Siedepunktes des eingesetzten Alkohols, empfohlen wird, woraus eine Durchführung in Druckgefäßen resultiert (DE-OS 2 740 243 = EP 1082; DE-OS 2 740 251 = EP 1083).

Hierbei verläuft die Umsetzung normalerweise nur bis zur Gleichgewichtseinstellung der Umesterungsreaktion. Nach der Entnahme aus dem Druckbehälter muß das Reaktionsgemisch sehr rasch vom Katalysator entfernt werden, beispielsweise durch eine Flash-Destillation, damit sich nicht in Umkehrung der Bildungsreaktion, etwa beim Abdestillieren des leichter siedenden Alkohols, die Ausgangsverbindungen zurückbilden. Beim Abdestillieren vom Katalysator kann sich das noch im Gleichgewicht befindliche Glykolcarbonat zu Kohlendioxid und zu Polyglykolen zersetzen, geht damit für eine weitere Umesterung unter Ausbeuteschmälerung verloren, und alle genannten Nebenprodukte stören die Aufarbeitung.

Aber auch wenn diese Abtrennung des Katalysators zufriedenstellend gelingt, müssen noch mehrere Destillationen durchgeführt werden. So ist zuerst eine Trennung von Hochsiedern (Glykolen und Glykolcarbonaten) von den Niedrigsiedern (Alkoholen und Dialkylcarbonaten) erforderlich. Die destillative Reinigung von Ethylenglykol, das beispielsweise für die Herstellung von Polyestern eine hohe Reinheit aufweisen muß, von unvollständig umgesetztem Ethylenglykolcarbonat, welches bevorzugt wiederum für die Herstellung der Dialkylcarbonate eingesetzt wird, ist jedoch nicht ohne Einschränkung möglich, da beide Verbindungen ein Azeotrop bilden. Eine ähnliche Schwierigkeit findet man vor, wenn man Methanol, welches die bevorzugte alkoholische Komponente ist, von dem gebildeten Dimethylcarbonat (DMC) abzutrennen hat. Auch diese Verbindungen bilden ein Azeotrop, das nur umständlich auseinander zu destillieren ist (EP 894 und darin zitierte Literatur).

Es bestand daher trotz vielfältiger Lösungsansätze ein dringendes Bedürfnis, die genannten vielfältigen Schwierigkeiten wirksam zu überwinden. Es wurde nun überraschend gefunden, daß man die genannten Nachteile bei der Umesterung von Glykolcarbonaten mit Alkoholen zu Dialkylcarbonaten beheben kann, wenn man die Umesterung in einer Kolonne im Gegenstrom der Ausgangsstoffe zueinander durchführt; hierbei kann bei unerwartet milden Bedingungen gearbeitet werden.

Die Erfindung betrifft daher ein Verfahren zur Herstellung von Dialkylcarbonaten der Formel

$(R^1O)_2CO$ (I),

in der

$R^1$ geradkettiges oder verzweigtes $C_1$-$C_4$-Alkyl bedeutet,

durch Umesterung von Ethylen- oder Propylencarbonat mit 3-30 Mol Alkoholen Formel

$R^1OH$ (II),

in der

$R^1$ die obige Bedeutung hat,

pro Mol Ethylen- bzw. Propylencarbonat in Gegenwart von Katalysatoren, das dadurch gekennzeichnet ist, daß man die Umesterung in einer mit Füllkörpern oder Einbauten ausgerüsteten Kolonne bei Temperaturen im Bereich von 60-160 °C durchführt und die Reaktionspartner im Gegenstrom so führt, daß das Ethylen- oder Propylencarbonat in den oberen Teil der Kolonne und der Alkohol in den unteren Teil der Kolonne eindosiert werden und der Katalysator in der Kolonne fest angeordnet ist oder in Lösung oder Suspension ebenfalls in den oberen Teil der Kolonne eindosiert wird, wobei das entstehende Dialkylcarbonat, gegebenenfalls im Gemisch mit Alkohol, am Kopf der Kolonne und das aus dem Ethylen- oder Propylencarbonat entstehende Ethylen- oder Propylenglykol, gegebenenfalls gemeinsam mit dem Katalysator, am Fuß der Kolonne entnommen werden.

Hierbei erreicht man trotz der milden Bedingungen eine vollständige Umesterung des Glykolcarbonats zum Dialkylcarbonat, so daß zunächst einmal das Auseinanderdestillieren des Glykolcarbonat-Azeotrops entfällt, da das Glykol schon frei von Glykolcarbonat am Fuß der Kolonne entnommen werden kann.

Infolgedessen wird die sonst übliche rasche Trennung des Reaktionsgemisches vom Katalysator überflüssig, da die Dialkylcarbonate am Kopf der Kolonne abgenommen werden, somit vom Glykol und vom Katalysator separiert sind und eine Umkehrung der Reaktion zu den Ausgangsprodukten nicht mehr möglich ist. Darüberhinaus kann bei einer Umesterung mit dem bevorzugt eingesetzten Methanol als Alkohol die Kolonne so betrieben werden, daß man am Kopf der Kolonne nicht nur das erwartete Azeotrop aus Methanol (etwa 70 %) und Dimethylcarbonat (etwa 30 %) erhält, sondern eine Mischung, die erheblich mehr an Dimethylcarbonat enthält, als dem Azeotrop entspricht. Es ist daher im Falle des Methanols sogar möglich, die Umesterung so durchzuführen, daß man als Ausgangsmaterial statt des reinen Methanols Gemische aus Methanol und Dimethylcarbonat, die gegebenenfalls bei anderen Prozessen anfallen, ohne vorherige schwierige Trennung in die erfindungsgemäße Umesterung mit Glykolcarbonat einsetzt. Diese Möglichkeit ist selbstverständlich auch bei der Verwendung anderer Alkohole als des Methanols möglich. Durch diese Möglichkeit der Verwendung von Gemischen aus Alkoholen und Dialkylcarbonaten zur Umesterung statt der reinen Alkohole ist die oft schwierige und energieintensive Trennung der Gemische, die in der überwiegenden Anzahl Azeotrope darstellen, nicht mehr erforderlich. In solchen Gemischen weisen der Alkohol und das Dialkylcarbonat den gleichen Alkylrest auf, wodurch unerwünschte, komplizierte Reaktionsgemische mit unterschiedlichen Alkylresten vermieden werden.

Diese Lösung der gestellten Aufgabe zeichnet sich durch ihre Einfachheit und ihre Eleganz aus.

Die einzusetzende Kolonne stellt im einfachsten Fall ein isotherm beheiztes, mit üblichen, für Destillationen zu verwendenden Füllkörpern gefülltes Rohr dar, dem am Kopf die Katalysatorlösung und das Glykolcarbonat aufgegeben werden. Diesem Gemisch wird von unten in Dampfform der zu verwendene Alkohol entgegengeschickt. Im Rohr werden überraschend schnell die Umesterungsschritte durchlaufen, so daß selbst bei einer relativ kurzen Kolonne dieser einfachen Bauart am Kopf beträchtliche Mengen Dialkylcarbonat übergehen.

Die Kolonne kann jedoch auch am unteren Ende einen bei höherer Temperatur arbeitenden Abtriebsteil enthalten, in welchem eine weitgehende bis vollständige Separierung des Alkohols aus dem herabrieselnden Glykol und Rückführung in den Umesterungsbereich der Kolonne erfolgt.

Weiterhin kann die Kolonne im oberen Teil einen bei niedrigerer Temperatur arbeitenden Verstärkerteil besitzen, um die Trennung von gasförmigem Alkohol und Dialkylcarbonat von Höhersiedern, wie z.B. Glykol und Glykolcarbonat, zu vervollkommnen und so ein hochprozentiges oder reines Gemisch aus Alkohol und Dialkylcarbonat am Kolonnenkopf zu entnehmen.

Die Energiezufuhr kann über den dampfförmig in die Kolonne eingebrachten Alkohol und/oder über den Sumpfverdampfer erfolgen. Der Alkohol kann gegebenenfalls auch flüssig eindosiert werden, dann muß die Energiezufuhr über den Sumpfverdampfer erfolgen. Im ersten Fall kann im Mittelteil der Kolonne, in der der größte Teil der Umesterung abläuft, eine Erweiterung des Kolonnendurchmessers bis auf das Vierfache der restlichen Teile von Vorteil sein. Im zweiten Fall muß die Verdampfungsenthalpie für den Alkohol durch den Abtriebsteil transportiert werden und führt hier zu einer hohen Gas- und Flüssigkeitsbelastung. Daraus resultiert eine Aufweitung der Kolonne im Abtriebsteil um die dort vorgesehenen Trennungen zu gewährleisten. Die Aufweitung und Länge des Abtriebsteils hängen von den gewählten Kolonneneinbauten im Abtriebsteil ab, und diese kann vom Fachmann ausgelegt werden.

Da im Laufe der Umesterung in der Gasphase zwei Moleküle Alkohol durch ein Molekül Dialkylcarbonat ersetzt werden, kann zur Konstanthaltung der Gasgeschwindigkeit im Mittelteil der Kolonne eine Querschnittsflächenverringerung bis zum Faktor 2 von Vorteil sein.

Somit kann die Kolonne sowohl isotherm beheizt werden als auch in bevorzugter Weise mit einer oder mehreren vom Hauptteil verschiedenen Temperaturzonen ausgestattet sein, wobei sich ein Temperaturgradient mit von oben nach unten steigenden Werten ergibt.

Die zu verwendenden Füllkörper bzw. geordneten Packungen sind die für Destillationen an sich üblichen, wie sie beispielsweise in Ullmann's Encyclopädie der Techn. Chemie, 4. Auflage, Band 2, S. 528 ff. oder in den Firmenschriften der in Frage kommenden Apparatebaufirmen beschrieben sind. Beispielsweise seien genannt: Raschig- oder Pallringe, Berl-, Intalex- oder Torussättel, Interpackkörper aus veschiedenen Materialien, wie Glas, Steinzeug, Porzellan, Kohlenstoff, Edelstahl, Kunststoff, die insbesondere bei der Verwendung von Metall gewebe- oder maschenartig verarbeitet sein können. Bevorzugt sind Füllkörper und geordnete Packungen, die eine große Oberfläche und eine gute Benetzung sowie eine ausreichende Verweilzeit der Flüssigkeit aufweisen. Dies sind beispielsweise: Pall- und Novolax-Ringe, Berlsättel, BX-Packungen, Montz-Pak, Mellapak, Melladur, Kerapak und CY-Packungen.

Für das erfindungsgemäße Verfahren sind jedoch nicht nur Füllkörperkolonnen geeignet, sondern auch solche mit festen Einbauten. Hierunter sind solche mit Glocken- bzw. Ventilböden mit hohen Verweilzeiten bei gutem Stoffaustausch bevorzugt.

Geeignet sind jedoch allgemein auch weitere Bodenkolonnen, z.B. solche mit Sieb-, Glocken-, Ventil-, Tunnel- und Zentrifugalböden, die wiederum in unterschiedlichen Ausführungen vorliegen können.

Die Kolonne wird so betrieben, daß man in die obere Hälfte, bevorzugt in das obere Drittel, eine Lösung des Katalysators im eingesetzten Glykolcarbonat oder auch in dem zugrundeliegenden Glykol oder auch im einzusetzenden Alkohol oder auch in einem systemfremden anderen geeigneten inerten Lösungsmittel eindosiert Für den Fall, daß ein in den Reaktionspartnern unlöslicher Katalysator eingesetzt wird, kann dieser auch im Gemisch mit den genannten Füllkörpern oder als Schüttung auf eingebauten Kolonnenböden eingesetzt werden. Ebenfalls in den oberen Bereich, bevorzugt in das obere Drittel der Kolonne, wird das Glykolcarbonat aufgegeben; es hat bevorzugt die gleiche Temperatur, die an dieser Stelle in der Kolonne herrscht. In die untere Hälfte der Kolonne, bevorzugt oberhalb einer gegebenenfalls vorhandenen Abtriebszone, wird der Alkohol eindosiert, in der Regel in Dampfform.

Am Kopf der Kolonne wird, bevorzugt nach Passieren einer Verstärkerzone, das Dialkylcarbonat abgenommen und kondensiert. Es enthält im allgemeinen noch Anteile des im System befindlichen Alkohols. Aus dem Sumpf der Kolonne trägt man ein bei sorgfältig eingestellten Bedingungen hochprozentiges Glykol aus, welches in einer Reindestillation von Katalysator und Verunreinigungen getrennt werden kann.

Das Molverhältnis in der Kolonne variiert von 3-30 Mol, bevorzugt 4-20 Mol, besonders bevorzugt 6-16 Mol Alkohol pro Mol des Ethylen- bzw. Propylencarbonats.

Die Temperatur in der Kolonne beträgt 60-160 °C, bevorzugt 60-150 °C, besonders bevorzugt 65-130 °C. Ein in bevorzugter Weise anzulegender Temperaturgradient liegt im angegebenen Temperaturbereich und steigt vom Kolonnenkopf zum Kolonnensumpf.

In der Regel wird die Reaktion des erfindungsgemäßen Verfahrens bei Normaldruck durchgeführt. Es ist jedoch auch möglich, bei schwach erhöhtem Druck bis zu etwa 3 bar, bevorzugt bis zu 2 bar oder bei erniedrigtem Druck bis hinab zu 50 mbar, bevorzugt bis zu 100 mbar, besonders bevorzugt bis zu 200 mbar zu arbeiten. In einer dem Fachmann bekannten Weise kann durch Abweichen vom Normaldruck eine Beeinflussung des etwa am Kopf zu entnehmenden Azeotrops möglich werden.

Die Raum-Zeit-Belastung der Kolonne liegt bei 0,1-3 g Gesamtmenge der Reaktionsteilnehmer pro ml wirksames Kolonnenvolumen pro Stunde, bevorzugt bei 0,2-2,5 g/ml/h, besonders bevorzugt bei 0,3-2,0 g/ml/h; das wirksame Kolonnenvolumen ist hierbei das der Füllkörperpackung oder das Volumen, in welchem sich feste Einbauten befinden. Katalysatoren, die für das erfindungsgemäße Verfahren in Frage kommen, sind in der Literatur bekannt.

Solche Katalysatoren sind beispielsweise Hydride, Oxide, Hydroxide, Alkoholate, Amide oder Salze von Alkalimetallen, wie Lithium, Natrium, Kalium, Rubidium und Cäsium, bevorzugt von Lithium, Natrium und Kalium, besonders bevorzugt von Natrium und Kalium (US-3 642 858, US-3 803 201, EP 1082). Für den Fall des Einsatzes der Alkoholate können diese erfindungsgemäß auch in situ durch Einsatz der elementaren Alkalimetalle und dem erfindungsgemäßen umzusetzenden Alkohol gebildet werden. Salze der Alkalimetalle können solche von organischen oder anorganischen Säuren sein, wie von Essigsäure, Propionsäure, Buttersäure, Benzoessäure, Stearinsäure, Kohlensäure (Carbonate oder Hydrogencarbonate), von Salzsäure, Bromwasserstoff- oder Iodwasserstoffsäure, Salpetersäure, Schwefelsäure, Fluorwasserstoffsäure, Phosphorsäure, Blausäure, Rhodanwasserstoffsäure, Borsäure, Zinnsäure, $C_1$-$C_4$-Stannonsäuren oder Antimonsäuren. In bevorzugter Weise kommen als Verbindungen der Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt.

Solche Alkalimetallverbindungen (gegebenenfalls in situ gebildet aus den freien Alkalimetallen) werden in Mengen von 0,001 bis 2 Gew.-%, bevorzugt 0,005 bis 0,9 Gew.-%, besonders bevorzugt 0,01 bis 0,5 Gew.-%, bezogen auf das umzusetzende Reaktionsgemisch, eingesetzt.

Es ist erfindungsgemäß möglich, solchen Alkalimetallverbindungen gegebenenfalls komplexierende Stoffe zuzusetzen (EP 274 953). Beispielsweise seien genannt Kronenether wie Dibenzo-18-krone-6, Polyethylenglykole oder bicyclische stickstoffhaltige Kryptanden.

Solche Komplexiermittel werden in Mengen von 0,1 bis 200 mol-%, bevorzugt 1 bis 100 mol-%, bezogen auf die Alkalimetallverbindung, eingesetzt. Als Katalysatoren für das erfindungsgemäße Verfahren sind ferner Thallium-I- und

Thallium-III-Verbindungen geeignet, wie die Oxide, Hydroxide, Carbonate, Acetate, Bromide, Chloride, Fluoride, Formiate, Nitrate, Cyanate, Stearate, Naphthenate, Benzoate, Cyclohexylphosphonate, Hexahydrobenzoate, das Cyclopentadienylthallium, Thalliummethylat, Thalliumethylat, bevorzugt Tl-(I)-oxid, Tl-(I)-hydroxid, Tl-(I)-carbonat, Tl-(I)-acetat, Tl-(III)-acetat, Tl-(I)-fluorid, Tl-(I)-formiat, Tl-(I)-nitrat, Tl-(I)-naphtenat und Tl-(I)-methylat (EP 1083). Die Mengen an Thalliumkatalysator sind nicht besonders kritisch. Sie betragen im allgemeinen 0,0001-10 Gew.-%, bevorzugt 0,001-1 Gew.-%, bezogen auf das gesamte

Reaktionsgemisch.

Im erfindungsgemäßen Verfahren können weiterhin stickstoffhaltige Basen als Katalysatoren eingesetzt werden (US-4 062 884). Genannt seien beispielsweise sek. oder tert- Amine wie Triethylamin, Tributylamin, Methyldibenzylamin, Dimethylcyclohexylamin u.a..

Die erfindungsgemäß eingesetzten Mengen der stickstoffhaltigen Basen liegen von 0,01 bis 10 Gew.-%, bevorzugt von 0,1 bis 5 Gew.-%, besonders bevorzugt von 0,1 bis 1 Gew.-%, bezogen auf das gesamte Reaktionsgemisch.

Weiterhin sind im erfindungsgemäßen Verfahren heterogene Katalysatorsysteme einsetzbar (US 4 062 884, US 4 691 041, JA 63/238 043, EP 298 167). Solche Systeme sind beispielsweise Ionentauscherharze mit funktionellen Gruppen aus tert. Aminen, quartären Ammoniumgruppen, wobei als Gegenionen Hydroxid, Chlorid oder Hydrogensulfat beispielsweise genannt seien, Sulfonsäuregruppen oder Carboxylgruppen, wobei für beide als Gegenionen Wasserstoff, Alkalimetalle oder Erdalkalimetalle beispielhaft genannt seien. Diese funktionellen Gruppen können entweder direkt oder über inerte Ketten an das Polymer gebunden sein. Weiterhin genannt seien Alkali- oder Erdalkalisilikate, imprägniert auf Siliciumdioxidträgern, sowie Ammonium-ausgetauschte Zeolithe.

Diese heterogenen Katalysatoren werden erfindungsgemäß bevorzugt stationär eingesetzt, es ist jedoch auch möglich, sie z.B. als feines Pulver in Suspension anzuwenden. In stationärer Form können die Katalysatoren z.B. anstelle der beschriebenen Füllkörper oder im Gemisch mit diesen eingesetzt werden.

Als Katalysatoren sind erfindungsgemäß ferner Verbindungen aus der Gruppe der Phosphine, Stibine, Arsine oder der zweiwertigen Schwefel- und Seien-Verbindungen sowie deren Oniumsalze einsetzbar (EP 180 387, US 4 734 518). Beispielhaft seien die folgenden genannt: Tributylphosphin, Triphenylphosphin, Diphenylphosphin, 1,3-Bis-(diphenylphosphino)propan, Triphenylarsin, Trimethylarsin, Tributylarsin, 1,2-Bis-(diphenylarsino)ethan, Triphenylstibin, Diphenylsulfid, Diphenyldisulfid, Diphenylselenid, Tetraphenylphosphoniumhalogenid (Cl, Br, J), Tetraphenylarsoniumhalogenid (Cl, Br, J), Triphenylsulfoniumhalogenid (Cl, Br) usw.

Die erfindungsgemäßen Einsatzmengen dieser Katalysatorengruppe liegen im Bereich von 0,1 bis 10 Gew.-%, bevorzugt von 0,1 bis 5 Gew.-%, besonders bevorzugt im Bereich von 0,1 bis 2 Gew.-%, bezogen auf das gesamte Reaktionsgemisch.

Weiterhin erfindungsgemäß einsetzbar sind Komplexe oder Salze des Zinns, Titans oder Zirkoniums (US 4 661 609). Beispiele solcher Systeme sind Butylstannonsäure, Zinnmethoxid, Dimethylzinn, Dibutylzinnoxid, Dibutylzinndilaurat, Tributylzinnhydrid, Tributylzinnchlorid, Zinn(II)ethylhexanoate, Zirkoniumalkoxide (Methyl, Ethyl, Butyl), Zirkonium(IV)halogenide (F, Cl, Br, J), Zirkoniumnitrate, Zirkoniumacetylacetonat, Titanalkoxide (Methyl, Ethyl, Isopropyl), Titanacetat, Titanacetylacetonat usw..

Die erfindungsgemäß einsetzbaren Mengen betragen 0,1 bis 10 Gew.-%, bevorzugt 1 bis 5 Gew.-%, bezogen auf das Gesamtgemisch.

Im erfindungsgemäßen Verfahren können weiterhin bifunktionelle Katalysatoren der Formel

$$[A_aX_b]_m \cdot [B_cY_d]_n \quad (III),$$

eingesetzt werden. In diesen bifunktionellen Katalysatoren wird das molare Verhältnis der beiden in eckigen Klammern stehenden Komponenten durch die Indices m und n ausgedrückt. Diese Indices können unabhängig voneinander Werte von 0,001-1, bevorzugt 0,01-1, besonders bevorzugt 0,05-1 und ganz besonders bevorzugt 0,1-1 annehmen. Innerhalb der eckigen Klammern stehen neutrale Salze aus je einem Kation und einem Anion. Die Indices a und b stellen unabhängig voneinander ganze Zahlen von 1-5 dar; die Indices c und d bedeuten unabhängig voneinander ganze Zahlen von 1-3, wobei den Forderungen der Valenzen der Kationen und Anionen zur Bildung solcher neutraler Salze zu entsprechen ist. Des weiteren bedeuten in (III)

A das Kation eines Metalles, das der
dritten Periode und Gruppe IIa, der
vierten Periode und Gruppe IIa, IVa-VIIIa, Ib oder IIb, der
fünften Periode und Gruppe IIa, IVa-VIIa, IIb oder IVb bzw. der
sechsten Periode und Gruppe IIa-VIa des Periodensystems der Elemente in der Kurzperiodenform angehört.

Die in Frage kommenden Metalle für das Kation A entnimmt der Fachmann den üblichen Darstellungen des Periodensystems der Elemente (Mendelejew) in der Kurzperiodenform. In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe

der genannten Metalle in Frage, wie beispielsweise Titanyl $TiO^{++}$ und Chromyl $CrO_2^{++}$.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, Iodid, Nitrat, das Anion einer Alkancarbonsäure mit 1-18 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (III) sind: Fluorid, Chlorid, Bromid, Iodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat und Laurinat. Besonders bevorzugte Anionen X sind: Chlorid, Bromid, Iodid, Acetat, Laurinat, Stearat, Palmitat, Decanoat, Nitrat und Sulfat.

Als Kation B in den Katalysatoren der Formel (III) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien hierbei genannt: das Lithium-, Natrium-, Kalium-, Rubidium-, Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kationen B solche der Formeln

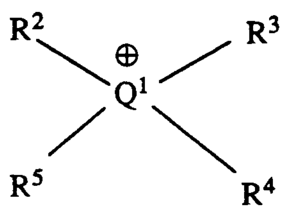

(IV) oder

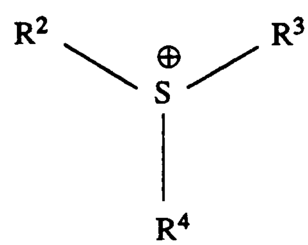

(V)

in Frage, worin

$Q^1$ für N, P, As oder Sb steht und

$R^2$, $R^3$, $R^4$ und $R^5$ unabhängig voneinander geradkettiges oder verzweigtes $C_1$–$C_{18}$-Alkyl oder $C_7$-$C_{12}$-Aralkyl sind und einer der Reste $R^2$-$R^5$ auch $C_6$-$C_{12}$-Aryl sein kann.

In besonders bevorzugter Weise ist B ein Kation der Formel

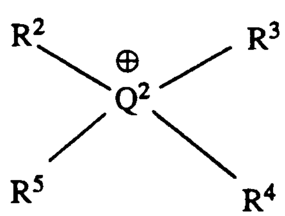

(VI)

worin,

$Q^2$ für N oder P, bevorzugt für N steht.

In ganz besonders bevorzugter Weise treten im Rahmen der Formeln (IV) bzw. (VI) an die Stelle der Reste $R^2$, $R^3$, $R^4$ und $R^5$ die Reste $R^{12}$, $R^{13}$, $R^{14}$ bzw. $R^{15}$, die unabhängig voneinander geradkettiges oder verzweigtes $C_1$-$C_{12}$-Alkyl oder $C_7$-$C_8$-Aralkyl bedeuten und einer der Reste $R^{12}$ bis $R^{15}$ auch Phenyl sein kann. In weiterhin ganz besonders bevorzugter Weise treten an die Stelle der Reste $R^{12}$, $R^{13}$, $R^{14}$ bzw. $R^{15}$, die Reste $R^{22}$, $R^{23}$, $R^{24}$ bzw. $R^{25}$, die unabhängig voneinander $C_1$-$C_8$-Alkyl oder Benzyl bedeuten und einer der Reste $R^{22}$ bis $R^{25}$ auch Phenyl sein kann.

Geradkettiges oder verzweigtes $C_1$-$C_{18}$-Alkyl ist beispielsweise Methyl, Ethyl, Propyl, Isopropyl, Butyl, Isobutyl, Hexyl, Octyl, Dodecyl, Hexadecyl oder Octadecyl. Bevorzugtes Alkyl hat 1-12 C-Atome, besonders bevorzugtes Alkyl hat 1-8 C-Atome.

$C_7$-$C_{12}$-Aralkyl ist beispielsweise Benzyl, Phenylethyl, Phenylpropyl, Naphthylmethyl oder Naphthylethyl; bevorzugtes Aralkyl ist Benzyl oder Phenylethyl, ganz besonders bevorzugtes Aralkyl ist Benzyl.

$C_6$-$C_{12}$-Aryl ist beispielsweise Phenyl, Naphthyl oder Biphenylyl, bevorzugt Phenyl.

Das Anion Y im Katalysator der Formel (III) ist ein Halogenidion, wie Fluorid, Chlorid, Bromid oder Iodid, bevorzugt Bromid oder Iodid, besonders bevorzugt Iodid. Es kann jedoch auch die Bedeutung von anderen, unter X genannten Anionen besitzen, wenn im konkreten Fall das Anion X Bromid oder Iodid ist.

Der bifunktionelle Katalysator der Formel (III) wird in einer Menge von 0,005-5 Gew.-%, bevorzugt 0,01-3 Gew.-%, besonders bevorzugt 0,01-1 Gew.-%, bezogen auf das gesamte Umesterungsgemisch, eingesetzt.

Diese Katalysatormengen unterscheiden sich teilweise von den in der Literatur genannten Mengen. Besonders überraschend ist, daß im erfindungsgemäßen Verfahren relativ hohe Konzentrationen an den wirksamen Katalysatoren auf der Basis von Alkaliverbindungen eingesetzt werden können, ohne daß es dabei zu den ausbeutemindernden und die Reaktionsführung behindernden Entwicklungen von $CO_2$ und zur Bildung von Polyolen kommt, wie dies beispielsweise aus DE-OS 2 740 243 und der dort zitierten Literatur und aus DE-OS 2 740 251 bekannt ist. Auch dies ist eine überraschende Besonderheit des erfindungsgemäßen Verfahrens.

Beispiele 1-7

In eine mit Raschigringen aus Glas gefüllte isotherm thermostatisierte Kolonne von 250 cm Länge und 30 mm Durchmesser dosierte man etwa 10 cm unterhalb des oberen Kolonnenendes separat eine Lösung des Katalysators KOH in Ethylenglykol und das Ausgangsprodukt Ethylenglykolcarbonat. Von unten schickte man diesem Strom dampfförmig Methanol entgegen, das man als Dampf etwa 30 cm oberhalb des unteren Kolonnenendes einspeiste. Am Kopf der Kolonne, die ohne Verstärkerteil arbeitete, nahm man ein Gemisch von Methanol und Dimethylcarbonat ab, am unteren Ende, das keinen Abtriebsteil besaß, Ethylenglykol, das gegebenenfalls noch untergeordnete Mengen an Ethylenglykolcarbonat und Alkohol enthielt.

Die folgende Tabelle 1 zeigt einige Beispiele für das erfindungsgemäße Umesterungsverfahren und deren Resultate. Diese wurden nach Einstellen konstanter Bedingungen und Verhältnisse in der Kolonne bestimmt. Da weder Abtriebs- noch Verstärkerteil vorhanden waren, enthielt der Sumpf noch Methanol und gegebenenfalls kleine Mengen Dimethylcarbonat und das Destillat noch Spuren Ethylenglykol bzw. Ethylenglykolcarbonat.

Beispiel 8

Das Verfahren der Beispiele 1 bis 7 wurde wiederholt und anstatt Methanol ein Strom an dampfförmigem Ethanol etwa 30 cm oberhalb des unteren Kolonnenendes eingespeist. Das Ergebnis zeigt die folgende Tabelle 2.

Tabelle 1: Beispiele 1-7

| Nr. | EGC g/h | Kat. g/h | MeOH g/h | Temp. °C | Destillat[1)] g/h | Destillat[1)] % MeOH | Destillat[1)] % DMC | Sumpf[2)] g/h | Sumpf[2)] % EG | Sumpf[2)] % EGC | Sumpf[2)] % MeOH |
|---|---|---|---|---|---|---|---|---|---|---|---|
| 1 | 101 | 0,7 | 330 | 80 | 302 | 64,9 | 34,4 | 131 | 53,5 | 0,3 | 45,5 |
| 2 | 202 | 1,4 | 660 | 80 | 599 | 66,9 | 32,1 | 259 | 53.3 | 1,2 | 44,2 |
| 3 | 195 | 1,4 | 970 | 100 | 735 | 73.3 | 25,6 | 427 | 30,4 | 4,5 | 63,2 |
| 4 | 170 | 0,7 | 660 | 70 | 457 | 62,6 | 36,9 | 366 | 33,2 | 1,7 | 63,7 |
| 5 | 250 | 1,4 | 1050 Azeotrop 70 % MeOH 30 % DMC | 80 | 930 | 57,4 | 42,1 | 370 | 36,7 | 30,8 | 22,8 +9,1 DMC |
| 6 | 202 | 1,4 | 630 | 80 | 450 | 54,2 | 45,3 | 380 | 37,4 | <0,1 | 61,2 |
| | Kolonne von 330 cm Länge | | | | | | | | | | |
| 7 | 200 | 1,3[3)] | 660 | 80 | 603 | 67,2 | 31,7 | 252 | 54,3 | 1,6 | 42,7 |

EGC = Ethylenglykolcarbonat
DMC = Dimethylcarbonat
MeOH = Methanol
EG = Ethylenglykol

1) und 2) Bei dieser Arbeitsweise ohne Abtriebs- und Verstärkerteil können
1) im Destillat noch Spuren EGC und EG
2) im Sumpf noch geringe Mengen DMC vorhanden sein.
3) Katalysator Na-methylat statt KOH

Tabelle 2: Beispiel 8

| Nr. | EGC g/h | Kat. KOH g/h | EtOH g/h | Temp. °C | Destillat[1] g/h | Destillat[1] % EtOH | Destillat[1] % DEC | Sumpf[2] g/h | Sumpf[2] % EG | Sumpf[2] % EGC | Sumpf[2] % EtOH |
|---|---|---|---|---|---|---|---|---|---|---|---|
| 8 | 104 | 2,8 | 358 | 100 | 149 | 78,7 | 20,2 | 310 | 37,7 | 26,9 | 22,2 |

EGC = Ethylenglykolcarbonat
DEC = Diethylcarbonat
EtOH = Ethanol
EG = Ethylenglykol

[1]und[2]
Bei dieser Arbeitsweise ohne Abtriebs- und Verstärkerteil können
[1]im Destillat noch Spuren EGC und EG
[2]im Sumpf noch geringe Anteile DEC enthalten sein.

Beispiel 9

Auf eine Kolonne von 28 mm Durchmesser und 120 cm Länge, die mit Füllkörpern aus Maschendrahtgeweben (Durchmesser 3 mm) gefüllt, auf 80 °C temperiert und am unteren Ende zusätzlich mit einem 40

cm langen auf 100 °C beheizten Abtriebsteil ausgerüstet war, gab man von oben pro Stunde 44 g EGC und 0,06 g KOH (in 3 g EG gelöst) und blies von unten, direkt oberhalb des Abtriebsteiles, 120 g 100 °C heißen MeOH-Dampf in die Kolonne ein.

Wenn sich die Kolonne im Gleichgewicht befand, wurde am Kopf ein Gemisch von 68,4 % MeOH und 31,6 % DMC erhalten und aus dem Sumpf ein Gemisch ausgetragen, das 81,0 % EG, 18,9 % MeOH und 0,1 % EGC enthielt.

Beispiel 10

Dosierte man in die Kolonne aus Beispiel 9 stündlich 88 g EGC, 240 g MeOH und 0,12 g KOH, in 6 g EG gelöst, ein, so erhielt man im stationären Zustand ein Kopfprodukt aus 60,6 % MeOH und 39,4 % DMC und ein Sumpfprodukt aus 71,5% EG, 21,8% MeOH und 5,8% EGC.

Erhöhte man die Temperatur im Abtriebsteil um 10°, so wurde der Gehalt an EGC im Sumpf auf 0,5 % reduziert.

Diese Beispiele zeigen zuerst einmal, wie überraschend glatt und rasch mit über 99 % Selektivität die Umesterung bei milden Bedingungen von 70-100° ohne Druckanwendung verläuft (vgl. dazu Beispiel 9 der EP 1082 oder Beispiel 6 und Vergleichsbeispiele der EP 1083 mit vorliegenden Beispielen).

Weiterhin läßt sich die Umesterung so lenken, daß im Sumpf praktisch kein EGC mehr vorhanden ist, also eine Trennung des EG/EGC-Azeotrops eingespart wird (siehe Beispiele 1, 6, 9, 11, 12 und 13), was nach bisherigem Stand der Technik nicht möglich war. Schließlich kann man ein Kopfprodukt erhalten, das mehr DMC erhält als dem MeOH/DMC-Azeotrop entspricht (Beispiele 1, 4, 5, 6 und 8), und man kann statt des MeOH das Azeotrop des MeOH und DMC einsetzen, wobei eine beträchtliche weitere Umesterung erfolgt (Beispiel 6: zusätzlich zum eindosierten werden weitere ca. 1,6 Mol DMC gebildet).

Beispiele 11 - 13

In einer 10-bödigen, isotherm beheizten Glockenbodenkolonne von 68 cm Länge und 5 cm Durchmesser dosierte man am oberen Ende das Edukt Ethylencarbonat und separat eine Lösung des Katalysators KOH in Ethylenglykol ein. Am unteren Ende der Kolonne schickte man dem ablaufenden Strom Methanol als Dampf entgegen. Am Kopf der Glockenbodenkolonne war über der Dosiereinheit eine Vigreux-Kolonne von 15 cm Länge und 3,5 cm Durchmesser als Trenneinheit aufgesetzt. Am unteren Ende befand sich ein 30 cm langes Abtriebsteil von 3,5 cm Duchmesser, gefüllt mit 4 x 4 mm Glasraschigringen. Am Kopf der Vigreux-Trennkolonne nahm man den Kopfstrom ab, am unteren Ende des Abtriebsteils wurde der Sumpfstrom entnommen. Die folgende Tabelle zeigt einige Beispiele für das erfindungsgemäße Verfahren. Die Zusammensetzungen wurden nach dem Einstellen konstanter Bedingungen bestimmt.

Tabelle 3: Beispiele 11 - 13

| Nr. | EGC g/h | Kat[1] g/h | MeOH g/h | Temp.°C Kolonne | Temp.°C Abtriebsteile | Destillat[2] g/h | Destillat[2] % MeOH | Destillat[2] % DMC | Sumpf[3] g/h | Sumpf[3] % EG | Sumpf[3] % EGC | Sumpf[3] % MeOH |
|---|---|---|---|---|---|---|---|---|---|---|---|---|
| 11 | 200 | 1,4 | 660 | 80°C | 80°C | 644 | 66,0 | 33,9 | 215 | 71,4 | - | 28,0 |
| 12 | 200 | 1,4 | 660 | 80°C | 100°C | 680 | 68,6 | 31,3 | 179 | 84,3 | - | 15,0 |
| 13 | 202 | 1,4 | 660 | 80°C | 120°C | 702 | 70,2 | 29,6 | 158 | 94,5 | - | 4,8 |

EGC = Ethylencarbonat
DMC = Dimethylcarbonat
MeOH = Methanol
EG = Ethylenglykol

1) KOH, dosiert als 10% Lösung in Glykol
2) Durch die aufgesetzte Vigreuxkolonne ist das Destillat praktisch EGC- und EG-frei
3) Der Sumpf ist frei von DMC

## Patentansprüche

1. Verfahren zur Herstellung von Dialkylcarbonaten der Formel

   $(R^1O)_2CO$,

   in der
   $R^1$ geradkettiges oder verzweigtes $C_1$-$C_4$-Alkyl bedeutet,
   durch Umesterung von Ethylen- oder Propylencarbonat mit 3-30 Mol Alkoholen der Formel

   $R^1OH$,

   in der
   $R^1$ die obige Bedeutung hat,
   pro Mol Ethylen- bzw. Propylencarbonat in Gegenwart von Katalysatoren, dadurch gekennzeichnet, daß man die Umesterung in einer mit Füllkörpern oder Einbauten ausgerüsteten Kolonne bei Temperaturen im Bereich von 60-160 °C durchführt und die Reaktionspartner im Gegenstrom so führt, daß das Ethylen- oder Propylencarbonat in den oberen Teil der Kolonne und der Alkohol in den unteren Teil der Kolonne eindosiert werden und der Katalysator in der Kolonne fest angeordnet ist oder als Lösung oder Suspension ebenfalls in den oberen Teil der Kolonne eindosiert wird, wobei das entstehende Dialkylcarbonat, gegebenenfalls im Gemisch mit Alkohol, am Kopf der Kolonne und das aus dem Ethylen- oder Propylencarbonat entstehende Ethylen- oder Propylenglykol, gegebenenfalls gemeinsam mit dem Katalysator, am Fuß der Kolonne entnommen werden.

2. Verfahren nach Anspruch 1, dadurch gekennzeichnet, daß der Alkohol Methanol ist.

3. Verfahren nach Anspruch 1, dadurch gekennzeichnet, daß der Alkohol Ethanol ist.

4. Verfahren nach Anspruch 1, dadurch gekennzeichnet, daß die Raum-Zeit-Belastung der Kolonne 0,1-3 g/ml/h, bezogen auf die Gesamtmenge der Reaktionsteilnehmer, beträgt.

5. Verfahren nach Anspruch 1, dadurch gekennzeichnet, daß als Alkohol statt des reinen Alkohols ein Alkohol-Dialkylcarbonat-Gemisch eingesetzt wird, in welchem der Alkohol und das Dialkylcarbonat den gleichen Alkylrest aufweisen.

6. Verfahren nach Anspruch 1, dadurch gekennzeichnet, daß die Kolonne einen Temperaturgradienten mit von oben nach unten steigenden Werten im angegebenen Bereich aufweist.

7. Verfahren nach Anspruch 1, dadurch gekennzeichnet, daß der Mittelteil der Kolonne eine Erweiterung bis auf das Vierfache des Durchmessers des restlichen Teils der Kolonne aufweist.

8. Verfahren nach Anspruch 1, dadurch gekennzeichnet, daß die Umesterung mit 4-20 Mol Alkoholen der Formel $R^1OH$ pro Mol Ethylen- bzw. Propylencarbonat durchgeführt wird.

9. Verfahren nach Anspruch 1, dadurch gekennzeichnet, daß bei Temperaturen im Bereich von 60-150 °C gearbeitet wird.

10. Verfahren nach Anspruch 4, dadurch gekennzeichnet, daß die Raum-Zeit-Belastung der Kolonne 0,2-2,5 g/ml/h beträgt.

## Claims

1. Process for the preparation of dialkyl carbonates of the formula

   $(R^1O)_2CO$ (I)

   in which

$R^1$ denotes straight-chain or branched $C_1$-$C_4$-alkyl,

by transesterification of ethylene carbonate or propylene carbonate with 3-30 mol of alcohols of the formula

$R^1OH$ (II)

in which

$R^1$ has the above meaning,

per mol of ethylene carbonate or propylene carbonate in the presence of catalysts, characterised in that the transesterification is carried out in a column equipped with packing or baffles at temperatures in the range of 60-160 °C, and the reactants are passed in countercurrent such that the ethylene carbonate or propylene carbonate are metered into the upper part of the column and the alcohol is metered into the lower part of the column and the catalyst is arranged as a fixed bed in the column or is also metered into the upper part of the column in solution or suspension, the dialkyl carbonate formed, if appropriate as a mixture with alcohol, being removed at the top of the column and the ethylene glycol or propylene glycol formed from the ethylene carbonate or propylene carbonate being removed at the foot of the column, if appropriate together with the catalyst.

**2.** Process according to Claim 1, characterised in that the alcohol is methanol.

**3.** Process according to Claim 1, characterised in that the alcohol is ethanol.

**4.** Process according to Claim 1, characterised in that the space/time loading of the column is 0.1-3 g/ml/hour, based on the total amount of the reaction participants.

**5.** Process according to Claim 1, characterised in that instead of the pure alcohol, an alcohol/dialkyl carbonate mixture in which the alcohol and the dialkyl carbonate have the same alkyl radical is employed as the alcohol.

**6.** Process according to Claim 1, characterised in that the column has a temperature gradient with values in the stated range which increase from the top downwards.

**7.** Process according to Claim 1, characterised in that the middle part of the column is widened to up to four times the diameter of the remaining part of the column.

**8.** Process according to Claim 1, characterised in that the transesterification is carried out with 4 - 20 mol of alcohols of the formula $R^1OH$ per mole of ethylene carbonate or propylene carbonate.

**9.** Process according to Claim 1, characterised in that it is carried out at temperatures in the range of 60 - 150 °C.

**10.** Process according to Claim 4, characterised in that the space/time loading of the column is 0.2 - 2.5 g/ml/h.

## Revendications

**1.** Procédé de préparation de carbonates de dialkyle de formule

$(R^1O)_2CO$ (I)

dans laquelle $R^1$ représente un groupe alkyle à chaîne droite ou ramifiée en $C_1$-$C_4$,

par transestérification du carbonate d'éthylène ou du carbonate de propylène par 3 à 30 mol d'alcools de formule

$R^1OH$ (II)

dans laquelle $R^1$ a les significations indiquées ci-dessus,

par mole de carbonate d'éthylène ou de propylène, en présence de catalyseurs, caractérisé en ce que

l'on réalise la transestérification dans une colonne contenant des corps de garnissage ou des éléments intérieurs à des températures dans l'intervalle de 60 à 160 °C, et on fait passer les réactifs à contre-courant, le carbonate d'éthylène ou de propylène étant introduit dans la partie supérieure de la colonne et l'alcool dans la partie inférieure de la colonne, le catalyseur est en disposition fixe dans la colonne ou également introduit, à l'état de solution ou de suspension, dans la partie supérieure de la colonne, le carbonate de dialkyle formé, éventuellement en mélange avec l'alcool, est évacué en tête de colonne, et l'éthylèneglycol ou propylèneglycol formé à partir du carbonate d'éthylène ou de propylène éventuellement avec le catalyseur, est évacué en pied de colonne.

**2.** Procédé selon la revendication 1, caractérisé en ce que l'alcool est le méthanol.

**3.** Procédé selon la revendication 1, caractérisé en ce que l'alcool est l'éthanol.

**4.** Procédé selon la revendication 1, caractérisé en ce que la charge spatiale horaire de la colonne est de 0,1 à 3 g/ml/h, pour la quantité totale des réactifs.

**5.** Procédé selon la revendication 1, caractérisé en ce que l'on met en oeuvre en tant qu'alcool, à la place de l'alcool pur, un mélange alcool-carbonate de dialkyle dans lequel l'alcool et le carbonate de dialkyle contiennent le même groupe alkyle.

**6.** Procédé selon la revendication 1, caractérisé en ce que la colonne présente un gradient de température avec des températures qui croissent du haut vers le bas dans l'intervalle spécifé.

**7.** Procédé selon la revendication 1, caractérisé en ce que la partie médiane de la colonne est élargie, dans une mesure allant jusqu'au quadruple du diamètre de la partie restante de la colonne.

**8.** Procédé selon la revendication 1, caractérisé en ce que la transistérification est réalisée avec 4 à 20 mol de l'alcool de formule $R^1OH$ par mole de carbonate d'éthylène ou de propylène.

**9.** Procédé selon la revendication 1, caractérisé en ce que l'on opère à des températures dans l'intervalle de 60 à 150 °C.

**10.** Procédé selon la revendication 4, caractérisé en ce que la charge spatiale horaire de la colonne est de 0,2 à 2,5 g/ml/h.